# EUROPEAN PATENT SPECIFICATION

(11) **EP 1 935 974 B1**
(45) Date of publication and mention of the grant of the patent: **27.08.2014**
(21) Application number: 06811987.4
(22) Date of filing: 12.10.2006
(51) Int. Cl.: C12M 3/00, C12M 1/12

(54) **CULTURE VESSEL AND CULTURE METHOD**
KULTURGEFÄSS UND KULTURVERFAHREN
CUVE DE CULTURE ET PROCÉDÉ DE CULTURE

(30) Priority: 14.10.2005 JP 2005299601
(43) Date of publication of application: 25.06.2008
(73) Proprietor: Toyo Seikan Kaisha, Ltd., Shinagawa-ku Tokyo 141-8640 (JP)
(72) Inventor: TANAKA, Satoshi, Yokohama-shi, Kanagawa, 240-0062 (JP); HATANO, Yasushi, Yokohama-shi, Kanagawa, 240-0062 (JP)
(74) Representative: Von Kreisler Selting Werner - Partnerschaft von Patentanwälten und Rechtsanwälten mbB
(86) International application number: PCT/JP2006/320798
(87) International publication number: WO 2007/043699

(56) References cited:
- WO-A2-02/42419
- WO-A2-03/103813
- JP-A- 04 020 281
- JP-A- 11 137 241
- JP-A- 11 137 241
- JP-A- 63 198 972
- JP-A- 2004 129 568
- JP-A- 2004 129 568
- JP-A- 2005 295 904
- JP-A- 2006 314 276
- JP-U- 01 099 200
- US-A1- 2001 024 821
- DATABASE WPI Week 199204 Thomson Scientific, London, GB; AN 1992-029677 XP002671780, & JP 3 277268 A (NISSHO CORP) 9 December 1991 (1991-12-09)

## Description

### Technical Field

This invention relates to a culture vessel and a method of culture and, more particularly, to a closed system type culture vessel and method of culture which have excellent gas exchange capability for oxygen and carbon dioxide and is suitable for culture of a cell or microorganism under a low humidity.

### Background Art of the invention

An incubator used generally for culture of a cell or microorganism has a very high inside humidity of 95% or over which causes a sanitary problem such as generation of mold or bacteria. This problem is particularly important in culture of a cell for medical use and hence control of humidity as well as control of sanitation must be made seriously. For this purpose, humidifying water to be stored in an incubator must be previously sterilized for preventing generation of mold or bacteria and, moreover, this sterilized water must be periodically supplemented. Even after putting the sterilized water in the incubator, it must be sterilized, if necessary, by irradiation of ultraviolet ray for maintaining the aseptic state of the humidifying water and, therefore, a ultraviolet ray irradiation equipment must be provided in the incubator. Thus, in the prior art culture of a cell or microorganism, the necessity for maintaining the inside of an incubator under a high humidity required troublesome controls of humidity and sanitation with resulting increase in the cost of culture.

For overcoming such problem on sanitation, various closed type culture vessels have been proposed and used. For example, Japanese Patent Application Laid-open Publication No. Sho 63-214178, Japanese Utility Model Application Laid-open Publication No. Hei 2-93999, Japanese Utility Model Application Laid-open Publication No. Hei 4-129800 and Japanese Patent Application Laid-open Publication No. Hei 3-277268 disclose culture bags which have a design of high gas permeability for material of the bag having regard to gas exchange capability of the bag for oxygen and carbon dioxide etc. These culture bags, however, have the problem that amount of culture medium is large and diffusion of gas in the culture medium follows a velocity control step with the result that there arises difference in dissolved gas concentration between a portion near the wall of the bag and a portion which is the remotest from the wall of the bag and an optimum dissolved gas concentration cannot be achieved. For this reason, these bags are inferior to the culture made in an open system in respect of efficiency of culture.

As a means for replacing the culture bags, there have been proposed and used, as disclosed in Japanese Patent Application Laid-open Publication No. 2004-129568 and Japanese Patent Application Laid-open Publication No. 2004-514432, rigid culture vessels of a thin and flat configuration having a gas permeable film. These thin and flat culture vessels have a large surface area to culture medium and therefore have excellent gas exchange capability. These vessels, however, generate an excessive amount of evaporating water vapor for use under a low humidity and have various problems which are caused by change in concentration of the culture medium component. That is, by evaporation of water, the concentration of a buffer solution in the culture medium becomes high and pH rises with resulting decrease in cell activity and decrease in the efficiency of culture and sometimes even damages to the cell.

As a buffer solution used in the culture medium, a HEPES buffer solution (N-2-hydroxiethylpiperazine-N'-2-ethanesulphonic acid buffer solution) or a buffer solution using Earle's salts or Hanks's salts, for example, may be used. These buffer solutions, however, are toxic to cells and hence the amount of addition of these solutions is restricted. High concentration of the culture medium brought about by evaporation of water therefore is undesirable. Further, concentration of the buffer solution used is restricted for it affects concentrations of calcium and magnesium and hence high concentration of the culture medium adversely affects cell culture. Furthermore, since concentration of waste materials such as lactic acid salt, urea nitrogen which is produced by the cell itself and obstruct culture becomes high, and since there is an allowable range in osmotic pressure of the cell, high concentration of the buffer system in the culture medium is undesirable.

When water vapor comes out, air takes the place of water vapor in the vessel and, as a result, air bubbles are generated. When these air bubbles burst, they are likely to cause damage to the cell. In a rigid vessel, a negative pressure may be produced in the vessel and this may also cause damage to the cell.

On the other hand, an amount of water evaporating from a culture vessel generally increases as temperature is elevated. This is partly because water vapor permeability of the material which constitutes the vessel becomes high but mainly because relative humidity becomes low. Comparing relative humidity at 25°C and that at 37°C, saturated water vapor pressure at 37°C becomes almost double that at 25°C and, therefore, if relative humidity at 25°C is 50%, relative humidity at 37°C becomes 25% which is half of relative humidity at 25°C. As a result, speed of evaporation becomes about 1.5 fold. In a case where humidity is not controlled, it is conceivable that such low humidity is brought about in the vicinity of 37°C and therefore it is important to consider this factor. However, there is no culture vessel in which such consideration has been given.

Accordingly, in the prior art culture vessel, it is generally a prerequisite to use it under a high humidity at 37°C and there is no vessel which has been designed having regard to use under a low humidity. In the above described publications, description is made about gas permeability coefficient, water vapor permeability and material of a gas permeable film used in a vessel, but there is no description whatsoever about a concept having regard to area of film and capacity of a culture vessel of a gas permeable film for using the vessel under a low humidity, or to what extent an amount of evaporating water vapor should be restricted.

### Disclosure Indication of the Invention

The present invention has been made in view of the above described problems of the prior art culture vessel. The present invention has considered that it is more advantageous in all aspects including sanitation, cost and management to conduct culture under a low humidity than to conduct culture under a high humidity in which strict humidity control and sanitation control are required. It is an object of the invention to provide a culture vessel having a gas permeable film in a part thereof which has high gas exchange capability and in which change of concentration due to water evaporation of culture medium can be confined within a range in which no problem will be produced even under a low humidity and therefore culture of a cell or microorganism for a long period of time becomes possible. It is another object of the invention to provide a method of culture using such culture vessel.

A culture vessel achieving the object of the invention comprises a gas permeable film which has oxygen permeability of P_{O2}×S/V>2.0, carbon dioxide permeability of P_{CO2}×S/V>8.0 and water vapor permeability of P_{H2O}×S/V<0.02 where V (ml) is a set capacity, S (cm²) is area of the gas permeable film, P_{O2} (ml/cm² · day · atm) is oxygen permeability at 37°C, P_{CO2} (ml/cm² day · atm) is carbon dioxide permeability at 37°C, P_{H2O} (g/cm² · day) is water vapor permeability at 37°C.

In one aspect of the invention, there is provided a culture vessel having a generally flat configuration and having the gas permeable film at least on one side thereof.

A method of culture achieving the object of the invention comprises a step of conducting culture by using the culture vessel as defined in claim 1 or 2 in such a manner that the relation of P_{H2O}× S / m×t/24≦0.10 will be achieved under relative humidity of 50% or below where m (g) is weight of input culture medium and t (hr) is length of time of culture.

In one aspect of the method of the invention, an object of culture is an animal cell.

According to the culture vessel of the invention, it has high gas exchange capability for oxygen and carbon dioxide and it can confine change of concentration of culture medium due to evaporation of water within a range in which no problem will be caused under a low humidity in which strict humidity control or a troublesome sanitation control including periodical supply of sterilized water and sterilization of humidifying water in the incubator by irradiation of ultraviolet ray are not required and therefore cost of culture can be saved.

According to the method of culture of the invention, by conducting culture by using the above described culture vessel under relative humidity of 50% or below, change of concentration of culture medium due to evaporation of water can be confined within a range in which no problem will be caused under a low humidity in which strict humidity control or sanitation control are not required and therefore it is advantageous in sanitation, cost and management whereby culture over a long period of time can be made under optimum conditions.

### Brief Description of the Drawings

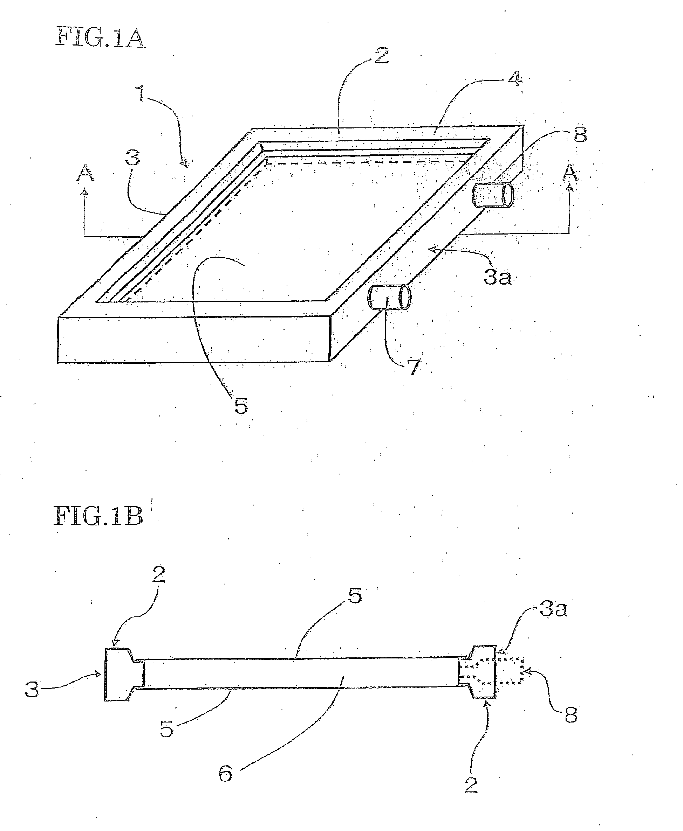

FIG. 1 shows a cell culture vessel of an embodiment of the present invention in which FIG. 1A is a perspective view and FIG. 1B is a sectional view along lines A - A in FIG. 1A.

### Description of Preferred Embodiments

Embodiments of the invention will be described below with reference the accompanying drawings.

The culture vessel according to the invention is a closed type vessel having a generally flat configuration made of a rigid material such as disclosed in the above described Japanese Patent Application Laid-open Publication No. 2004-129568 and Japanese Patent Application Laid-open Publication No. 2004-514432. This vessel has a pair of flat surfaces opposite to each other with a space between them and side portions between these flat surfaces. At least one of these flat surfaces has a gas permeable film.

FIG. 1 shows an embodiment of the culture vessel of the invention in which FIG. 1A is a perspective view and FIG. 1B is a sectional view along lines A-A in FIG. 1A.

A culture vessel 1 for a cell or the like is a vessel of a generally oblong, flat configuration is made of plastic having flexibility and rigidity to some extent such as polyethylene, polypropylene and vinyl chloride. A frame 2 is made by injection molding or other method and consists of long sides 3, short sides 4 and a window portion 5. On the front and back sides of the frame 2, there are formed, by a suitable means, gas permeable films 5, 5 face to face with a predetermined interval between them in the direction of thickness of the frame 2.

This frame 2 is of a stepped structure in such a manner that the outer peripheral side of the frame 2 is thick and the inner peripheral side thereof is thin. The outer peripheral side of the frame 2 is made thick for securing rigidity and also securing a necessary portion for attaching a member for filling and taking out contents liquid while the inner peripheral side which constitutes the window portion is adjusted to a thickness necessary for securing a set space for filling contents liquid.

The gas permeable films 5 are made of a material which allows passing of culture gas and does not allow passing of microorganism or the like which contaminates the cell. This material is selected from resins to be described later.

In this culture vessel 1, a space 6 (FIG. 1B) formed by the frame 2 and the gas permeable films 5, 5 formed on the front and back sides of the frame 2 constitutes a space for filling contents liquid therein and the capacity of this space 6 is the set capacity of the vessel 1.

A contents liquid filling tube 7 and a tube 8 for taking out contents liquid are attached to one long side 3a of the long sides 3 of the frame 2. Contents liquid which is a mixed liquid of a cell and a culture medium is filled from the contents liquid filling tube 7 into the vessel 1 and the vessel 1 is sealed with a suitable known means. After completion of filling of contents liquid and sealing, culture is carried out for a predetermined period of time at predetermined temperature, humidity, gas composition and concentration. After completion of the culture, contents liquid is taken out of the contents liquid take-out tube 8.

The vessel 1 may be a vessel in which the inside capacity can be changed. In this case, the set capacity V becomes variable but, since area S of the gas permeable film 5 is constant, even when the set capacity of the vessel 1 is changed according to the amount of medium to be filled such as the amount of cell to be cultured, culture can be made within a range of the determined volume which can be cultured.

The gas permeable film 5 should be made of a material which has excellent gas exchange capability for oxygen and carbon dioxide and has very small water vapor permeability so that it is suited for culture under a low humidity. The gas permeable film 5 must have oxygen permeability of P_{O2}×S/V>2.0, carbon dioxide permeability of P_{CO2}×S/V>8.0 and water vapor permeability of P_{H2O}×S/V<0.02 when P_{O2} is oxygen permeability at 37°C, P_{CO2} is carbon dioxide permeability at 37°C, P_{H2O} is water vapor permeability at 37°C.

The gas permeable film 5 is of a material in which oxygen permeability, carbon dioxide permeability and water vapor permeability are not affected by relative humidity. As a material satisfying such requirement, a monolayer or multilayer film consisting principally of a polyolefin resin or a fluoroplastic may preferably be used.

As materials of the gas permeable film satisfying such conditions may be cited homopolymer resins or copolymer resins polymerized from one or more olefinic monomers including olefins such as ethylene, propylene, butene, pentene, hexene, heptene and octene, and cyclic olefins such as norbornene and tetracyclododecene, e.g., polyolefins such as polyethylene, polypropylene, polybutene-1, poly-4-methyl-pentene-1, and cyclic olefin copolymer. As other materials may be cited polystyrene, fluoroplastic such as tetrafluoride ethylene-hexafluoride propylene copolymer and silicone polymer. A suitable material should be selected from these resins in accordance with the type of the cell to be cultured and culture conditions such as humidity of the incubator.

The gas permeable film 5 should preferably have a suitable tension so that it will not be slackened when it is provided on the frame and also have excellent transmissivity to visible ray.

### Examples

### Measurement

### 1. Set capacity of culture vessel

Set capacity V (ml) of the culture vessel is the amount of culture medium in the space defined by the gas permeable films and the frame in a state wherein the gas permeable films maintain equilibrium with the opposing film and the culture space is completely filled with the culture medium.

### 2. Area of the gas permeable films

The area of the gas permeable film S (cm²) is the area of the film which is calculated on the assumption that it is a flat film enclosed by the frame without taking into account minute projections or depressions on the surface of the film.

### 3. Oxygen permeability and carbon dioxide permeability

The oxygen permeability and carbon dioxide permeability were measured with the gas permeability measuring device (GPM-250: made by GL Sciences) at 37°C by using, as a gas supplied, a mixed gas of nitrogen (65%), oxygen (15%) and carbon dioxide (20%).

### 4. Water vapor permeability

The water vapor permeability was measured with the water vapor permeability measuring device (PERMATRAN-W3/30: made by Mocon) at 37°C.

### Evaluation

### 1. Rate of evaporation of the culture medium (contents liquid)

Suspension at a predetermined concentration consisting of the culture medium and cells was filled in the culture vessel by the set capacity V (ml) and then total weight Wo (g) of the vessel was measured. Weight w (g) of the dish by itself which was previously measured was deducted from the total weight Wo to calculate the initial filling weight (Wo - w) of the culture medium. After conducting culture, total weight W (g) of the vessel was measured in the same manner and this weight W was deducted from the total weight Wo of the vessel before conducting the culture to calculate weight of evaporated culture medium (Wo - W). The rate of evaporation of the culture liquid was obtained by dividing the evaporated weight of the culture medium (Wo - W) by the initial filling weight of the culture medium (Wo - w). i.e., (Wo - W)/(Wo - w).

### 2. Growth rate

After conducting the culture, the number of collected cells was counted and growth rate was calculated from the number of cells which were inoculated at starting of the culture. An example which exhibited growth rate of 90% or over relative to a cell (control) which was cultured under relative humidity of 100% was designated by the mark o and an example which exhibited growth rate of less than 90% as the mark x.

### Example 1

A frame made of high density polyethylene having long sides of 127 mm, short sides of 85 mm and thickness of 5.8 mm is made thin in its inside portion to form a window portion having long sides of 83.3 mm, short sides of 60 mm and thickness of 2 mm. On the front and back sides of the window portion of this frame, there were provided gas permeable films each of which was an extrusion film having thickness of 100 µm and consisting of a polypropyrene (pp) layer having thickness of 20 µm and a linear low density polyethylene (LLDPE) layer produced by using a metallocene type catalyst and having thickness of 80 µm. Each of the films was heat sealed with its LLDPE layer being disposed inside and thus the culture vessel shown in Table 1 and having the structure shown in Fig. 1 was provided.

Contents liquid of 10 ml of the set capacity in which mouse derived cytotoxic T-cell CTLL-2 was inoculated at a concentration of 4000 cells/ml in a serum-free culture medium (AlyS505N made by Saibo Kagaku Kenkyusho, containing 700IU/ml of IL-2) was filled in the vessel from the contents liquid filling tube and then the vessel was sealed.

Then, culture was conducted for 95 hours in an atmosphere at 37°C in which relative humidity was 20% and carbon diode concentration was 5% and the rate of evaporation of the culture liquid (contents liquid) and growth rate were evaluated. The results of the evaluation are shown in Table 2.

### Example 2

Culture was conducted and evaluation was made in the same manner as in Example 1 except that, as shown in Table 1, the gas permeable films of the culture vessel were made of TPX (made by Mitsui Kagaku, poly-4-methylpentene-1) having thickness of 30 µm and LLDPE having thickness of 70 µm consisting of a multi-layer film laminated with a urethane type bonding agent.

### Example 3

Culture was conducted and evaluation was made in the same manner as in Example 1 except that, as shown in Table 1, the gas permeable films of the culture vessel were made of FEP (made by Daikin Kogyou, tetrafluoroethylene · hexafluoropropyrene copolymer) having thickness of 30 µm and LLDPE having thickness of 70 µm consisting of a multi-layer film laminated with a urethane type bonding agent.

### Comparative Example 1

Culture was conducted and evaluation was made in the same manner as in Example 1 except that a frame made of polystyrene (PS) was used and, as shown in Table 1, the gas permeable films of the culture vessel were made of a PS film having thickness of 100 µm.

### Comparative Example 2

Culture was conducted and evaluation was made in the same manner as in Example 1 except that a culture bag was produced by sealing four sides of a LLDPE film having thickness of 100 µm so that an inside portion which was not heat sealed had long sides of 215 mm and short sides of 150 mm and this bag was used as the culture vessel shown in Table 1 by using the walls of this bag as permeable films.

### Comparative Example 3

Culture was conducted and evaluation was made in the same manner as in Example 1 except that the thickness of the window portion of the frame in Example 1 was made 6 mm so that the set capacity of the culture vessel was made triple as large as that of Example 1.

**Table 1**

| | culture vessel | | | | | | contents liquid weight(g) (cell· culture liquid) | culture time t(hr) |
|---|---|---|---|---|---|---|---|---|
| | set capacity V (ml) | gas permeable film (film structure) | | | | | | |
| | | material | area of film S (cm²) | oxygen permeability (P_{O2} × S/V) | carbon dioxide permeability (P_{CO2} × S/V) | P_{H2O} × S/V | | |
| Example 1 | 10 | LLDPE/PP | 100 | 2.8 | 9.9 | 0.0027 | 10 | 95 |
| Example 2 | 10 | LLDPE/TPX | 100 | 8.3 | 22.7 | 0.0036 | 10 | 95 |
| Example 3 | 10 | LLDPE/FEP | 100 | 5.1 | 14.9 | 0.0012 | 10 | 95 |
| Comparative Example 1 | 10 | PS | 100 | 1.6 | 6.4 | 0.0300 | 10 | 95 |
| Comparative Example2 | 350 | LLDPE | 645 | 1.2 | 4.2 | 0.0005 | 350 | 95 |
| Comparative Example3 | 30 | LLDPE/PP | 100 | 0.9 | 3.3 | 0.0009 | 30 | 95 |

| | | | | | | | | |
|---|---|---|---|---|---|---|---|---|
| 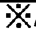 As oxygen permeability P_{O2}, carbon dioxide permeability P_{CO2} and water vapor permeability P_{H2}O, values at 37°C and relative humidity of 90% are used. | | | | | | | | |

**Table 2**

| Evaluation | | |
|---|---|---|
| P_{H2O}×S/m×t/24 Rate of evaporation of culture liquid (contents liquid) (%) | | growth rate |
| Example 1 | 1.07 | ○ |
| Example 2 | 1.43 | ○ |
| Example 3 | 0.48 | ○ |
| Comparative Example 1 | 11.88 | × |
| Comparative Example 2 | 0.19 | × |
| Comparative Example 3 | 0.36 | × |

As will be apparent from the results shown in Tables 1 and 2, the cell culture vessels of Examples 1 to 3 satisfy values of required properties of the present invention in all of oxygen exchange capability, carbon dioxide exchange capability and water vapor barrier capability and thereby exhibit that they are cell culture vessels which are suitable for cell culture under a low humidity.

In contrast, the cell culture vessel of Comparative Example 1 fails to attain the required properties of the present invention in all of oxygen permeability (oxygen exchange capability), carbon dioxide permeability (carbon dioxide capability) and water vapor permeability (water vapor barrier capability) and the cell culture vessels of Comparative Examples 2 and 3 fail to attain the required properties of the present invention in oxygen permeability (oxygen exchange capability) and carbon dioxide permeability (carbon dioxide exchange capability) and therefore it will be understood that they are not suitable as culture vessels under a low humidity.

The culture vessel of the present invention can be used also for culture of microorganisms and is particularly useful for culture of animal cells.

Culture is generally conducted at a temperature within a range from 35°C to 40°C in the case of animal cells and at a temperature within a range from 15°C to 60°C in the case of microorganisms such as *Escherichia coli.*

### Industrial Utility

The present invention can be applied to a method of culture and a culture vessel. Particularly, the present invention can be applied as a method of culture and a culture vessel which has high gas exchange capability for oxygen and carbon dioxide and which can confine change of concentration of culture medium due to evaporation of water within a range in which no problem will be caused under a low humidity in which strict humidity control or a troublesome sanitation control including periodical supply of sterilized water and sterilization of humidifying water in the incubator by irradiation of ultraviolet ray are not required and therefore cost of culture can be saved and therefore is suitable for culture of cells and microorganisms under a low humidity..

## Claims

1. A culture vessel **(1)** used for culture of a cell or microorganism under relative humidity of 50 % or below comprising **a frame (2) and** a gas permeable film **(5) wherein said gas permeable film (5)** has oxygen permeability of P₀₂ x S/V > **2.0,** carbon dioxide permeability of P_{CO2} x S/V > 8.0 and water vapor permeability of P_{H20} × S/V <0.02 where V(ml) **is the amount of culture medium in the space defined by the gas permeable films (5) and the frame (2) in a state wherein the gas permeable film (5) is parallel with the opposing film and the culture space is completely filled with the culture medium**, S (cm²) is area of the gas permeable film, P_{O2} (ml/cm² x day x atm) is oxygen permeability at 37°C, P_{CO2} (ml/cm² x day x atm) is carbon dioxide permeability at 37 °C, P_{H2O} (g/cm² x day) is water vapor permeability at 37 °C.

2. A culture vessel **(1)** as defined in claim 1 having a generally flat configuration and having the gas permeable film **(5)** at least on one side thereof.

3. A method of culture comprising a step of conducting culture by using the culture vessel **(1)** as defined in claim 1 or 2 in such a manner that the relation of P_{H2O}× S/m×t/24≦0.10 will be achieved under relative humidity of 50% or below where m (g) is weight of input culture medium and t (hr) is length of time of culture.

4. A method as defined in claim 3 wherein an object of culture is an animal cell.

## Patentansprüche

1. Kulturgefäß (1), das für die Kultur einer Zelle oder eines Mikroorganismus bei einer relativen Feuchtigkeit von 50% oder weniger verwendet wird, umfassend einen Rahmen (2) und eine gasdurchlässige Folie (5), wobei die gasdurchlässige Folie (5) eine Sauerstoffdurchlässigkeit von P_{O2} x S/V > 2,0, eine Kohlendioxiddurchlässigkeit von P_{CO2} x S/V > 8,0 und eine Wasserdampfdurchlässigkeit von P_{H2O} x S/V < 0,02 aufweist, wobei V (ml) die Menge des Kulturmediums in dem durch die gasdurchlässigen Folien (5) und den Rahmen (2) definierten Zwischenraum in einem Zustand ist, in dem die gasdurchlässige Folie (5) parallel zu der gegenüberliegenden Folie verläuft und der Kulturraum vollständig mit dem Kulturmedium gefüllt ist, S (cm²) die Fläche der gasdurchlässigen Folie ist, P_{O2} (ml/cm² x Tag x atm) die Sauerstoffdurchlässigkeit bei 37 °C ist, P_{CO2} (ml/cm² x Tag x atm) die Kohlendioxiddurchlässigkeit bei 37 °C ist, P_{H2O} (g/cm² x Tag) die Wasserdampfdurchlässigkeit bei 37 °C ist.

2. Kulturgefäß (1) gemäß Anspruch 1, das eine im Wesentlichen flache Konfiguration aufweist und die gasdurchlässige Folie (5) auf wenigstens einer Seite davon aufweist.

3. Kulturverfahren, umfassend einen Schritt des Durchführens einer Kultur unter Verwendung des Kulturgefäßes (1), wie es in Anspruch 1 oder 2 definiert ist, in einer solchen Weise, dass die Beziehung P_{H2O} x S/m x t/24 ≤ 0,10 bei einer relativen Feuchtigkeit von 50% oder weniger erfüllt wird, wobei m (g) das Gewicht des eingebrachten Kulturmediums ist und t (h) die Zeitdauer der Kultur ist.

4. Verfahren gemäß Anspruch 3, wobei das Kulturobjekt eine tierische Zelle ist.

## Revendications

1. Récipient de culture (1) utilisé pour cultiver une cellule ou un microorganisme à une humidité relative de 50% ou moins, comprenant un cadre (2) et une feuille perméable aux gaz (5), dans lequel ladite feuille perméable aux gaz (5) a une perméabilité à l'oxygène de P_{O2} x S/V > 2,0, une perméabilité au dioxyde de carbone de P_{CO2} x S/V > 8,0, et une perméabilité à la vapeur d'eau de P_{H2O} x S/V < 0,02, où V (ml) est la quantité du milieu de culture dans l'espace défini par les feuilles perméables aux gaz (5) et le cadre (2) dans un état dans lequel la feuille perméable aux gaz (5) est parallèle à la feuille opposée, et l'espace de culture est complètement rempli du milieu de culture, S (cm²) est la superficie de la feuille perméable aux gaz, P_{O2} (ml/cm² x jour x atm) est la perméabilité à l'oxygène à 37 °C, P_{CO2} (ml/cm² x jour x atm) est la perméabilité au dioxyde de carbone à 37 °C, P_{H2O} (g/cm² x jour) est la perméabilité à la vapeur d'eau à 37 °C.

2. Récipient de culture (1) tel que défini dans la revendication 1, ayant une configuration sensiblement plane, et portant la feuille perméable aux gaz (5) sur au moins un de ses côtés.

3. Procédé de culture, comprenant l'étape consistant à réaliser une culture en utilisant le récipient de culture (1) tel que défini dans la revendication 1 ou 2 d'une telle manière que la relation P_{H2O} x S/m x t/24 ≤ 0,10 soit satisfaite à une humidité relative de 50% ou moins, où m (g) est le poids du milieu de culture introduit et t (hr) est la durée de la culture.

4. Procédé tel que défini dans la revendication 3, dans lequel l'objet de culture est une cellule animale.
